# EUROPÄISCHE PATENTSCHRIFT

(11) **EP 0 956 361 B1**
(45) Veröffentlichungstag und Bekanntmachung des Hinweises auf die Patenterteilung: **14.05.2003**
(21) Anmeldenummer: 98951196.9
(22) Anmeldetag: 14.08.1998
(51) Int. Cl.: C12Q 1/00

(54) **VERFAHREN UND VORRICHTUNG ZUR ERFASSUNG DER TOXISCHEN UND MUTAGENEN WIRKUNG VON CHEMIKALIEN UND SUBSTANZGEMISCHEN**
METHOD AND DEVICE FOR DETECTING THE TOXIC AND MUTAGENIC EFFECT OF CHEMICALS AND MIXTURES OF SUBSTANCES
PROCEDE ET DISPOSITIF DE DETECTION DE L'EFFET TOXIQUE ET MUTAGENE DE PRODUITS CHIMIQUES ET DE MELANGES DE SUBSTANCES

(30) Priorität: 15.08.1997 DE 19736261
(43) Veröffentlichungstag der Anmeldung: 17.11.1999
(73) Patentinhaber: IUT Privates Institut für Umwelttoxikologie GmbH, 17489 Greifswald (DE)
(72) Erfinder: BERCHER, Horst, D-17493 Greifswald (DE)
(74) Vertreter: Wehlan, Helmut, Dr.
(86) Internationale Anmeldenummer: DE9802466
(87) Internationale Veröffentlichungsnummer: WO99009201

(56) Entgegenhaltungen:
- WO-A-92/15687
- FR-A- 2 683 632
- US-A- 5 786 162
- SIMPSON M L ET AL: "Bioluminescent-bioreporter integrated circuits form novel whole -cell biosensors" TRENDS IN BIOTECHNOLOGY, Bd. 16, Nr. 8, 1. Dezember 1998, Seite 332-338 XP004131479
- CHUN U ET AL: "Continuous pollution monitoring using Photobacterium phosphoreum" RESOURCES CONSERVATION AND RECYCLING, Bd. 18, Nr. 1, November 1996, Seite 25-40 XP004016613
- RAMANATHAN S ET AL: "Bacterial biosensors for monitoring toxic metals" TRENDS IN BIOTECHNOLOGY, Bd. 15, Nr. 12, Dezember 1997, Seite 500-506 XP004097428
- TOTHILL I E ET AL: "Developments in bioassay methods for toxicity testing in water treatment" TRAC, TRENDS IN ANALYTICAL CHEMISTRY, Bd. 15, Nr. 5, Mai 1996, Seite 178-188 XP004034762
- DATABASE WPI Derwent Publications Ltd., London, GB; AN 95-239034 XP002094790 & RU 2 025 466 A (KAUSHANSKII) , 30. Dezember 1994
- DATABASE WPI Derwent Publications Ltd., London, GB; AN 88-336565 XP002094791 & SU 1 395 673 A (MOSCOW LOMONOSOV UNIV.) , 15. Mai 1988

## Beschreibung

Die Erfindung betrifft ein Verfahren und eine Vorrichtung zur Erfassung der toxischen und mutagenen Wirkung von Chemikalien und Substanzgemischen mit Hilfe immobilisierter Leuchtbakterien oder mittels durch Transfer geeigneter Plasmid-Vektoren zur Biolumineszenz befähigter Mikroorganismen. Da die Erfassung der biologischen Wirkungen über einen längeren Zeitraum erfolgen kann, ermöglicht die Erfindung Überwachungsaufgaben in der Umwelt und eine Prozesskontrolle in der Landwirtschaft und in der Industrie.

Der Einsatz von Mikroorganismen und isolierten Zellen zum Nachweis toxischer oder mutagener Effekte ist bekannt. So wurden Bakterien (DE-OS 3902982; LÜMMEN, P.: forum mikrobiologie 10, 428-434 (1988)), isolierte Zellen (ROSSI, A. et al.: Pharmacol. & Toxicol. 68, 424-429 (1991)), Protozoen (BRINKMANN, G.: Z. Wasser- und Abwasser-Forsch. 11, 210-215 (1978)), pflanzliche Protoplasten (OVERMEYER, S. et al.: UWSF-Z. Umweltchem. Ökotox. 6, 5-8 (1994)) und Grünalgen (BRINKMANN, G. u. R.. Kühn: Z. Wasser- und Abwasser-Forsch. 10, 87-98 (1977) zum Nachweis toxischer Wirkungen von Schadstoffen eingesetzt. Es ist auch vorgeschlagen worden, bestimmte Enterobakterien (ODA, Y. et al.: Mutation Res. 147, 219-229 (1985)) und Leuchtbakterien (ULITZUR, S. et al.: Mutation Res. 74, 113-124 (1980)) zur Erfassung mutagener Effekte einzusetzen.

Diese bekannten Lösungen sind dadurch gekennzeichnet, dass sie sehr zeitaufwendig sind und in Speziallaboratorien durchgeführt werden müssen, nicht für Messungen unter Feldbedingungen geeignet sind und nur Einzelbestimmungen erlauben.

In der Schrift WO 92/15687 A werden fusionierte Gene und deren Verwendung für die Bestimmung der Anwesenheit von Metallen oder xenobiotischen Verbindungen beschrieben. Dabei wird die Bestimmung eines Metalls oder einer xenobiotischen Verbindungen in einem flüssigen Medium für einen Konzentrationsbereich von 1 bis 120 ppm mit lyophilisierten und immobilisierten Zellen durchgeführt. Im Dokument FR-A-2 683 632 wird ein Sensor mit immobilisierten Leuchtbakterien offenbart. Dieser Sensor dient zur Kontrolle der Toxizität unterschiedlich zusammengesetzter Abwässer. Eine Detektion von Luftschadstoffen ist in dieser Schrift nicht vorgesehen. Auch ist mit dem dort beschriebenen Verfahren keine Erfassung mutagener Effekte möglich.

Auf einem Gel immobilisierte Mikroorganismen sind Gegenstand der Offenlegungsschrift RU 2 025 466 A. In diesem Dokument wird über eine optoelektronische Komponente nicht berichtet.

Chun et al. (Resources conservation and recycling, Bd. 18, Nr. 1, Nov. 1996, S. 25-40) stellen ein Gerät zur Detektion von Biolumineszenz in marinen Bakterien vor. Chun et al. nutzen eine Vorrichtung mit einer Durchflusszelle und messen die Biolumineszenz der Bakterien mit einem Fluoreszenzpektrometer. Die Vorrichtung hat den Nachteil, dass durch das ständige Überströmen der Bakterien mit Schadstoffen in der Durchflusszelle die Bakterien stark beansprucht werden und ist eine Regeneration der Mikroorganismen nicht möglich ist.

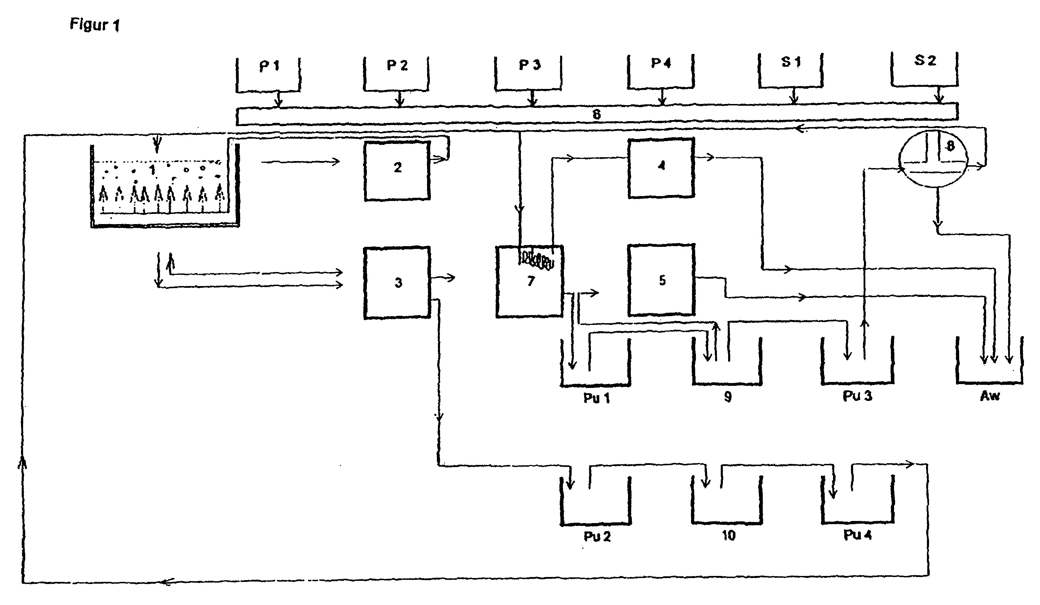

Der Erfindung liegt die Aufgabe zugrunde, ein Verfahren zu entwickeln, mit dem sich die Wirkung von Schadstoffen erfassen lässt. Die Aufgabe wurde dadurch gelöst, dass Leuchtbakterien oder durch den Transfer geeigneter Plasmid-Vektoren zur Biolumineszenz befähigte Mikroorganismen immobilisiert und die Immobilisate mit einer optoelektronischen Komponente zur Lichtmessung so versehen werden, dass ein Biosensor gebildet wird. Der erfindungsgemäße Biosensor wird in einem vom Prinzip her bekannten Fließinjektionssystem gemäß Figur 1 zur Steuerung der Probenzufuhr eingesetzt.

Die Immobilisierung der Leuchtbakterien oder durch den Transfer geeigneter Plasmid-Vektoren zur Biolumineszenz befähigter Mikroorganismen erfolgt mit einer geeigneten Immobilisierungsprozedur für Mikroorganismen, z.B.
1. der mechanische Einschluss der o.g. Mikroorganismen in eine für den Probendurchfluss geeignete Messzelle mittels einer porösen, schadstoffdurchlässigen Membran
2. der Einschluss in ein Gel, wie z.B. Agar, Kollagen oder Polyacrylamid
3. die Einkapselung, wie z.B. in Barium- bzw. Calciumalginat oder Zellulosesulfat
4. die Bindung an Oberflächen von Membranen oder von anderen Formteilen, gegebenenfalls unter Zuhilfenahme eines geeigneten Fixierungsmittels, wie z.B. Glutaraldehyd oder epoxidgruppenhaltige Substanzen.

Die optoelektronische Komponente des Biosensors besteht aus einem Messinstrument für die schwachen, emittierten Lichtsignale, wie z.B. einer Fotodiode oder einem Fotoelement und entsprechenden Verstärkern und Signalauswerteeinheiten sowie einem Anzeigeinstrument.

Die Kopplung der Immobilisate (biologischer Teil des Biosensors) mit der optoelektronischen Komponente erfolgt dergestalt, dass die Immobilisate entweder in eine wenigstens an bestimmten Teilen lichtdurchlässige Messzelle inkubiert werden, an deren lichtdurchlässigem Abschnitt das Lichtmessinstrument angeordnet und so die Biolumineszenz der Mikroorganismen messbar ist, oder bei Verwendung lichtundurchlässiger Messzellen die Biolumineszenzimpulse über einen Lichtleiter an das Lichtmessinstrument weitergeleitet werden. Die Probenzu- und abfuhr erfolgt durch den Einbau des erfindungsgemäßen Biosensors in ein vom Prinzip her bekanntes Fließinjektionssystem, das aus mehreren Pumpen und Magnetventilen besteht, die über einen Mikroprozessor oder einen externen Computer angesteuert werden (Figur 1).

Die Erfindung besteht in einer Kombination bekannter und neuer Merkmale, die sich gegenseitig beeinflussen und in ihrer neuen Gesamtwirkung den erstrebten Erfolg ergeben, der darin liegt, dass die Erfassung der biologischen Wirkungen von Chemikalien und Substanzgemischen über einen längeren Zeitraum erfolgen kann und Überwachungsaufgaben in der Umwelt und eine Prozesskontrolle in der Landwirtschaft und in der Industrie ermöglicht werden.

Der erfindungsgemäße Einsatz der immobilisierten Leuchtbakterien oder durch den Transfer geeigneter Plasmid-Vektoren zur Biolumineszenz befähigter Mikroorganismen ermöglicht überraschenderweise gegenüber den bekannten Lösungen neue Anwendungen. Beispiele für neue Anwendungen sind Überwachungsaufgaben in der Umwelt, wie die Überwachung von Luft, Oberflächenwasser, Abwasser oder Deponiesickerwasser und die Prozesskontrolle in der Landwirtschaft und der Industrie. Vorteilhaft gegenüber bekannten Lösungen ist auch die Einsatzmöglichkeit der immobilisierten Bakterien für mehrfache Einzelmessungen. Darunter ist die wiederholte Nutzung ein und derselben Immobilisate zur nachfolgenden Messung verschiedener Proben zu verstehen.

Beispiele für derartige neue Anwendungen bestehen in der Messung verschiedener Umweltproben und in einem Screening auf Toxizität und Mutagenität bei Chemikalien, Kosmetika, Arzneimitteln, Pestiziden, Bedarfsgegenständen und Lebensmitteln.

### Figur 1:

- 1: Behälter mit luftgesättigter Trägerflüssigkeit
- 2: Druckpumpe für Luft
- 3 - 5: Flüssigkeitspumpen
- 6: 6-Wegemagnetventil
- 7: Injektionsventil
- 8: 3-Wegemagnetventil
- 9: Messküvette mit Immobilisat
- 10: Vergleichsküvette mit Immobilisat
- P1 -P4: Probe 1 - 4
- S1 - S2: Standard 1 und 2
- Pu1 - Pu4: Puffer 1 - 4
- Aw: Abwasser

Die Erfindung soll anhand von Ausführungsbeispielen näher erläutert werden.

### Ausführungsbeispiele

Beispiel 1:
Spontane Dunkelvarianten von V. fischeri werden in einer Lösung (in g/l) von 5 g Pepton, 3 g Glycerol, 15,5 g NaCl, 0,75 g KCl, 12,3 g MgSO₄ x 7H₂O, 1,45 g CaCl₂ x 2H₂O, 0,075 g K₂HPO₄ x 2H₂O und 1,0 g NH₄Cl inkubiert. Die Bakteriendichte (ca, 10⁵ Zellen/ml) wird über eine Trübungsmessung und der pH auf 7,0 eingestellt. Mit physiologischer NaCl-Lösung wird eine 1.25 %ige Natriumalginatlösung hergestellt und steril filtriert. Dazu wird soviel der Bakteriensuspension gegeben, dass die Endkonzentration der Natriumalginatlösung 1 % beträgt. Diese Lösung wird mittels einer feinen Kanüle und unter konstantem Druck in ein Fällungsbad aus 0,2 M CaCl₂ getropft. Nach dem Erstarren des Alginats werden die Immobilisate mehrfach mit physiologischer Kochsalzlösung gewaschen und in eine Messkammer des Fließinjektionssystems nach Figur 1 gegeben. Bei Kontakt mit einer mutagen wirkenden Substanz werden die Bakterien zur Biolumineszenz angeregt. Eine Verdoppelung des emittierten Lichtes gegenüber einer Negativ-Kontrollmessung wird als Nachweis einer mutagen wirkenden Substanz gewertet. Durch vorherige Behandlung der Probe mit einer Leberenzymfraktion (S 9-Mix) lassen sich auch Komütagene erfassen.

Beispiel 2:
1,5 g Agar werden unter Erwärmung und Rühren in 100 ml destilliertem Wasser gelöst. Nach dem Abkühlen auf 40- 45 °C werden die Bakterien V. fischeri als Suspension (in 30 g NaCl, 6,10 g NaH₂PO₄ x 3H₂O, 0,204 g MgSO₄ x 7H₂O, 0,50 g (NH₄)₂HPO₄, 3 ml Glycerol, 5 g Pepton, 0,50 g Hefeextrakt mit H₂O auf 1 l aufgefüllt, eingestellt mit NaOH oder HCl auf pH 7,2 +/- 0,2) in die warme Lösung gegeben. Anschließend lässt man die Mischung als Schrägagar in der Messkammer des Fließinjektionssystems erstarren. Bei Kontakt mit einer Probe, die toxische Substanzen enthält, wird über eine Stoffwechselhemmung die Biolumineszenz der Leuchtbakterien eingeschränkt. Eine 20 %ige Hemmung der Biolumineszenz wird als toxische Wirkung gewertet.

Beispiel 3:
5 ml einer Suspension von V. fischeri (siehe Beispiel 2) werden mit Eis gekühlt. In 5 ml einer eisgekühlten 0,2 M Kaliumphosphat-Pufferlösung (pH 7,0) werden 1,63 g Acrylamid, 0,08 g N,N-Methylen-Bisacrylamid und 5 mg Ammoniumperoxodisulfat gegeben und bis zur Auflösung der Feststoffe unter Kühlung gerührt. Hinzu werden die Bakteriensuspension und 0,08 ml N,N,N',N'-Tetramethylethylendiamin unter Rühren getropft. Die Polymerisation ist nach etwa 60 bis 90 Minuten abgeschlossen. Das fertige Gel wird mit der Pufferlösung gewaschen und in der Messzelle des Fließinjektionssystems inkubiert. Mit diesen Immobilisaten lässt sich analog Beispiel 2 die toxische Wirkung von Chemikalienproben nachweisen.

Beispiel 4:
E.coli-Bakterien, in die mittels Standardtechnik der LUX-Genkomplex übertragen wurde, werden, wie unter Beispiel 3 beschrieben, immobilisiert und zur Messung der toxischen Wirkung eingesetzt.

Beispiel 5:
4%iges Na-Zellulosesulfat in einer Lösung nach Beispiel 1 wird in ein 2%iges Fällbad aus Polydiallyldimethylammoniumchlorid in 0,9%iger NaCl-Lösung eingetropft und dort 8-10 min. ausgehärtet. Durch Überführung der erhaltenen Kugeln in eine hypotonische Lösung bilden sich stabile, licht- und schadstoffdurchlässige Kapseln, in deren Hohlraum mit Hilfe einer sehr feinen Kanüle Leuchtbakteriensuspensionen wie z.B. der Arten V. fischeri, V. harvei, Ph. phosphoreum oder Ph. leiognathi oder Suspensionen anderer zur Biolumineszenz befähigter Mikroorganismen eingebracht werden können.

Diese Kapseln werden in der Messkammer des Fließinjektionssystems zur Erfassung toxischer Wirkungen von Substanzengemischen oder Einzelsubstanzen eingesetzt.

Beispiel 6:
In Leuchtbakterien- oder anderen Mikroorganismensuspensionen nach Beispiel 1 und 5 wird 2% Na-Alginat vollständig gelöst. Durch Eintauchen steriler Glasfaserfilter-Plättchen in diese Lösung und anschließende Inkubation der Filter in 0,2M CaCl₂-Lösung und mehrfaches Waschen in 0,9%iger NaCl-Lösung erhält man stabile Glasfaser-Alignat-Immobilisate, welche in der Messzelle des Biosensors zum Einsatz kommen.

Beispiel 7:
In einer Zellsuspension wie in Beispiel 1 beschrieben, wird 4% Na-Zellulosesulfat vollständig gelöst. Mit Hilfe einer Spritze und durch Variation des Kanülendurchmessers können nach Fällung in einer Polydiallyldimethylammoniumchlorid-Lösung und Waschen in 0,9%iger NaCl-Lösung stabile Zellulosesulfat-Kapseln verschiedenen Durchmessers erhalten werden.

Diese Immobilisierungstechnik ist sowohl für verschiedene Wildstämme und Dunkelvarianten von Leuchtbakterien als auch für andere zur Biolumineszenz befähigte Mikroorganismen geeignet.

## Patentansprüche

1. Verfahren zur Erfassung der toxischen und mutagenen Wirkung von Chemikalien und Substanzgemischen, **dadurch gekennzeichnet, dass** in einem Fließinjektionssystem gemäß Figur 1, bestehend aus mehreren Pumpen (2-5), Ventilen (6-8), Küvetten (9,10) und Behältern (1, P1-P4, S1,S2, Pu1-Pu4, Aw), immobilisierte, zur Biolumineszenz befähigte Organismen mit einer optoelektronischen Komponente zur Lichtmessung so versehen werden, dass ein Biosensor gebildet wird.

2. Verfahren nach Anspruch 1, **dadurch gekennzeichnet, dass** als Organismen Leuchtbakterien oder durch einen Transfer geeigneter Plasmid-Vektoren zur Biolumineszenz befähigte Mikroorganismen eingesetzt werden.

3. Verfahren nach Anspruch 1 und 2, **dadurch gekennzeichnet, dass** die Leuchtbakterien oder die durch einen Transfer geeigneter Plasmid-Vektoren zur Biolumineszenz befähigten Mikroorganismen in Natriumalginat gelöst und mittels Glasfaserfilterplättchen und Calciumchlorid in stabile Glasfaser-Alginat-Immobilisate überführt werden.

4. Verfahren nach Anspruch 1 bis 3, **dadurch gekennzeichnet, dass** zur Erfassung der mutagenen Wirkung von Chemikalien und Substanzgemischen spontane Dunkelvarianten von V. fischeri eingesetzt werden.

5. Verfahren nach Anspruch 1 uns 2, **dadurch gekennzeichnet, dass** zur Bestimmung der toxischen Wirkung von Chemikalien und Substanzgemischen Leuchtbakterien oder durch einen Transfer geeigneter Plasmid-Vektoren zur Biolumineszenz befähigte Mikroorganismen in ein Gel oder Kapseln eingeschlossen werden.

6. Verfahren nach Anspruch 5, **dadurch gekennzeichnet, dass** E.coli-Bakterien, in die der LUX-Genkomplex übertragen wurde, eingesetzt werden

7. Verfahren nach den Ansprüchen 5 und 6, **dadurch gekennzeichnet, dass** es für mehrfache Einzelmessungen eingesetzt wird.

8. Vorrichtung zur Erfassung der toxischen und mutagenen Wirkung von Chemikalien und Substanzgemischen, bestehend aus einem Biosensor, der seinerseits aus immobilisierten, zur Biolumineszenz befähigten Organismen und einer optoelektronischen Komponente zur Lichtmessung gebildet wird und in ein Fließinjektionssystem gemäß Figur 1, das mehrere Pumpen (2-5), Ventile (6-8), Küvetten (9,10) und Behälter (1, P1-P4, S1,S2, Pu1-Pu4, Aw) enthält, integriert ist.

9. Vorrichtung nach Anspruch 8, **dadurch gekennzeichnet, dass** die Organismen Leuchtbakterien oder durch einen Transfer geeigneter Plasmid-Vektoren zur Biolumineszenz befähigte Mikroorganismen darstellen.

10. Verwendung der Vorrichtung nach Anspruch 8 und 9 für Überwachungsaufgaben in der Umwelt, wie Luft, Oberflächenwasser, Abwasser oder Deponiesickerwasser und der Prozeßkontrolle in der Landwirtschaft und der Industrie.

11. Verwendung nach Anspruch 10 zur Messung von Umweltproben und in einem Screening auf Toxizität und Mutagenität bei Chemikalien, Kosmetika, Arzneimitteln, Pestiziden, Bedarfsgegenständen und Lebensmitteln.

## Claims

1. A method of detecting the toxic and mutagenic effect of chemicals and substance mixtures, **characterized in that** in a flow injection system according to Figure 1 comprised of a plurality of pumps (2-5), valves (6-8), cuvettes (9, 10) and vessels (1, P1-P4, S1, S2, Pu1-Pu4, Aw), immobilized organisms that have been rendered bioluminescent are provided with an optoelectronic component for photometry so that a biosensor is formed.

2. The method according to claim 1, **characterized in that** luminous bacteria or microorganisms that have been rendered bioluminescent by a transfer of adequate vector plasmids are used as said organisms.

3. The method according to claims 1 and 2, **characterized in that** said luminous bacteria or said microorganisms that have been rendered bioluminescent by a transfer of adequate vector plasmids are dissolved in sodium alginate, and are converted into stable glass fiber-alginate immobilisates by means of glass fiber filter plates and calcium chloride.

4. The method according to claims 1 through 3, **characterized in that** , spontaneous dark variants of V. fischeri are used for detecting the mutagenic effect of chemicals and substance mixtures.

5. The method according to claim 1 and 2, **characterized in that** luminous bacteria or microorganisms that have been rendered bioluminescent by a transfer of adequate vector plasmids are enclosed in a gel or in capsules for determining the toxic effect of chemicals and substance mixtures.

6. The method of claim 5, **characterized in that** E. coli bacteria are used, into which the LUX gene complex has been transferred.

7. The method according to claims 5 and 6, **characterized in that** it is used for multiple single measurements.

8. An apparatus for detecting the toxic and mutagenic effect of chemicals and substance mixtures, comprised of a biosensor which is formed from immobilized organisms that have been rendered bioluminescent and an optoelectronic component for photometry, and which is integrated within a flow injection system according to Figure 1 that includes a plurality of pumps (2-5), valves (6-8), cuvettes (9, 10) and vessels (1, P1-P4, S1, S2, Pu1-Pu4, Aw).

9. The apparatus according to claim 8, **characterized in that** said organisms are luminous bacteria or microorganisms that have been rendered bioluminescent by a transfer of adequate vector plasmids.

10. Use of the apparatus according to claims 8 and 9 for surveillance tasks in the environment, such as air, surface water, waste water or leachate from landfills, and in agricultural and industrial process controls.

11. The use according to claim 10 for measuring environmental samples and in screening for toxicity and mutagenicity in chemicals, cosmetics, medicinal preparations, pesticides, consumer goods and food.

## Revendications

1. Procédé d'acquisition de l'effet toxique et mutagène de produits chimiques et de mélanges de substances **caractérisé en ce que** dans un système à flux continu selon la Figure 1, constitué de plusieurs pompes (2 à 5), de valves (6 à 8), de cuves (9, 10) et de récipients (1, P1 à P4, S1, S2, Pu1 à Pu4, Aw), des organismes immobilisés ayant été pourvus de capacités de bioluminescence sont équipés d'un composant optoélectronique pour mesurer la lumière de manière telle qu'un capteur biologique soit constitué.

2. Procédé selon la revendication 1, **caractérisé en ce qu'**en tant qu'organismes, sont utilisés des bactéries lumineuses ou des micro-organismes auxquels des capacités de bioluminescence ont été conférées par un transfert de plasmagènes adaptés.

3. Procédé selon les revendications 1 et 2, **caractérisé en ce que** les bactéries lumineuses ou les micro-organismes auxquels des capacités de bioluminescence ont été conférées par un transfert de plasmagènes adaptés sont dissous dans de l'alginate de sodium et transformés au moyen de plaquettes de filtre de fibre de verre et de chlorure de calcium en un système d'alginate/fibre de verre stable.

4. Procédé selon les revendications 1 à 3, **caractérisé en ce que** pour acquérir l'effet mutagène de produits chimiques et de mélanges de substances, des variantes sombres spontanées de V. fischeri sont employées.

5. Procédé selon les revendications 1 et 2, **caractérisé en ce que** pour déterminer l'effet toxique de produits chimiques et de mélanges de substances, des bactéries lumineuses ou des micro-organismes auxquels des capacités de bioluminescence ont été conférées par un transfert de plasmagènes adaptés sont inclus dans un gel ou dans des capsules.

6. Procédé selon la revendication 5 **caractérisé en ce que** des bactéries E. coli, auxquelles a été transféré le complexe de gènes LUX, sont employées.

7. Procédé selon les revendications 5 et 6, **caractérisé en ce qu'**il est mis en oeuvre pour de multiples mesures individuelles.

8. Dispositif d'acquisition de l'effet toxique et mutagène de produits chimiques et de mélanges de substances, se constituant d'un capteur biologique formé de son côté d'organismes immobilisés auxquels des capacités de bioluminescence ont été conférées et d'un composant optoélectronique pour la mesure de la lumière et qui est intégré dans un système à flux continu selon la Figure 1 qui contient plusieurs pompes (2 à 5), des valves (6 à 8), des cuves (9, 10) et des récipients (1, P1 à P4, S1, S2, Pu1 à Pu4, Aw).

9. Dispositif selon la revendication 8, **caractérisé en ce que** les organismes sont des bactéries lumineuses ou des micro-organismes auxquels des capacités de bioluminescence ont été conférées par un transfert de plasmagènes adaptés.

10. Utilisation du dispositif selon les revendications 8 et 9 pour des tâches de surveillance de l'environnement, tel l'air, l'eau de surface, l'eau d'écoulement ou l'eau d'infiltration de décharge, et le contrôle de processus dans l'agriculture et l'industrie.

11. Utilisation selon la revendication 10 pour la mesure d'échantillons environnementaux et dans une recherche de toxicité et de caractéristique mutagène de produits chimiques, de cosmétiques, de médicaments, de pesticides, de produits d'usage courant et d'aliments.
